# EUROPÄISCHE PATENTSCHRIFT

(11) **EP 3 692 138 B1**
(45) Veröffentlichungstag und Bekanntmachung des Hinweises auf die Patenterteilung: **15.12.2021**
(21) Anmeldenummer: 18782698.7
(22) Anmeldetag: 01.10.2018
(51) Int. Cl.: C12N 5/071, C12N 5/079

(54) **MIKROPHYSIOLOGISCHE ORGANOIDKULTUR**
MICRO-PHYSIOLOGICAL ORGANOID CULTURE
CULTURE MICROPHYSIOLOGIQUE D'ORGANOÏDES

(30) Priorität: 05.10.2017 DE 102017217738
(43) Veröffentlichungstag der Anmeldung: 12.08.2020
(73) Patentinhaber: Eberhard Karls Universität Tübingen, 72074 Tübingen (DE)
(72) Erfinder: LOSKILL, Peter, 70199 Stuttgart (DE); PROBST, Christopher, 70186 Stuttgart (DE); LIEBAU, Stefan, 72414 Rangendingen (DE); ACHBERGER, Kevin, 72074 Tübingen (DE); HADERSPECK, Jasmin, 72074 Tübingen (DE)
(74) Vertreter: Wohlfahrt, Jan Günther
(86) Internationale Anmeldenummer: PCT/EP2018/076645
(87) Internationale Veröffentlichungsnummer: WO 2019/068640

(56) Entgegenhaltungen:
- LAURA C. BAHLMANN ET AL: "DYnamic bioengineered hydrogels as scaffolds for advanced stem cell and organoid culture", MRS COMMUNICATIONS, Bd. 7, Nr. 03, 29. August 2017 (2017-08-29), Seiten 472-486, XP055523881, ISSN: 2159-6859, DOI: 10.1557/mrc.2017.72
- KEVIN S. JACKSON ET AL: "Three-Dimensional Ovarian Organ Culture as a Tool to Study Normal Ovarian Surface Epithelial Wound Repair", ENDOCRINOLOGY, Bd. 150, Nr. 8, 1. August 2009 (2009-08-01), Seiten 3921-3926, XP055523875, US ISSN: 0013-7227, DOI: 10.1210/en.2008-1674
- M. A. LANCASTER ET AL: "Organogenesis in a dish: Modeling development and disease using organoid technologies", SCIENCE, Bd. 345, Nr. 6194, 17. Juli 2014 (2014-07-17), Seiten 283,1-9, XP055130123, ISSN: 0036-8075, DOI: 10.1126/science.1247125
- DODSON KIRSTEN H ET AL: "Retina-on-a-chip: a microfluidic platform for point access signaling studies", BIOMEDICAL MICRODEVICES, KLUWER, DORDRECHT, NL, Bd. 17, Nr. 6, 11. November 2015 (2015-11-11), Seiten 1-10, XP035902050, ISSN: 1387-2176, DOI: 10.1007/S10544-015-0019-X [gefunden am 2015-11-11]
- JULIA ROGAL ET AL: "Integration concepts for multi-organ chips: how to maintain flexibility?!", FUTURE SCIENCE OA, Bd. 3, Nr. 2, 1. Mai 2017 (2017-05-01), Seite FSO180, XP055527584, DOI: 10.4155/fsoa-2016-0092
- LLONCH SÍLVIA ET AL: "Organoid technology for retinal repair", DEVELOPMENTAL BIOLOGY, Bd. 433, Nr. 2, 25. Dezember 2017 (2017-12-25), Seiten 132-143, XP085322057, ISSN: 0012-1606, DOI: 10.1016/J.YDBIO.2017.09.028
- HARSPECK J., ACBERGER K., PROBST C., ROGAL J., ET AL.: "Development of a 3-dimensional microphysiological Retina-on-a-chip system.", INVESTIGATIVE OPHTALMOLOGY & VISUAL SCIENCE, Bd. 59, Nr. 9, 29. April 2018 (2018-04-29) , - 3. Mai 2018 (2018-05-03), XP002786886, Gefunden im Internet: URL:https://iovs.arvojournals.org/article. aspx?articleid=2689245> [gefunden am 2018-11-20]
- CHRISTOPHER PROBST ET AL: "High-throughput organ-on-a-chip systems: Current status and remaining challenges", CURRENT OPINION IN BIOMEDICAL ENGINEERING, Bd. 6, 1. Juni 2018 (2018-06-01), Seiten 33-41, XP055523859, ISSN: 2468-4511, DOI: 10.1016/j.cobme.2018.02.004

## Beschreibung

Die vorliegende Erfindung liegt auf dem Gebiet der Kultivierung von biologischen Zellen und Geweben mit organähnlicher Funktion in mikrophysiologischen Maßstab und stellt ein Verfahren zur mikrophysiologischen Co-Kultivierung von 3D-Organoid-Gewebe und mindestens einer 2D-Zellschicht bereit.

Zell- und Stammzell-basierte in vitro Modelle werden entwickelt, welche ethisch problematische und kostenintensive Tiermodelle bei der Erforschung genetischer oder idiopathischer Erkrankungen des menschlichen Körpers und bei der Entwicklung prophylaktischer und therapeutischer Wirkstoffe dagegen ersetzen können.

Mikrophysiologische (MPS) oder so genannte "Organ-on-a-Chip" (OoaC) Systeme ermöglichen die Kultivierung von körpereigen Zellen wie Zelllinien, Primärzellen, embryonalen Ursprungs oder induziert pluripotenten Stammzellen (iPSZ) unter physiologischen Bedingungen, um spezifische Gewebe wie Lunge, Herz, Darm, Niere nachzubilden. Komplexe Stammzell-basierte Organsysteme aus mehreren Zelltypen wurden entwickelt, so genannte Organoide. Diese entstehen während der in vitro Differenzierung weitgehend selbstständig und selbstorganiserend unter dem Einfluss weniger externer Signalmoleküle. Beispiele dafür sind Pankreas-, Darm-, Gehirn- oder Retina-Organoide. Sie sind in der Lage bis zu einem gewissen Grad physiologische Zusammenhänge simulieren, da sie sich selbstorganisierend zu komplexen Zellverbänden zusammenschließen. So können aus körpereigenen Stammzellen eines Patienten (individualisierte iPS-Zellen) spezifisch patientenspezifische in vitro Organoidsysteme bereitgestellt werden, die insbesondere zur Entwicklung einer individualisierten Therapie, Reihenuntersuchungen zu Wirkstoffwirkungen (drug screening) und Wirkstoffsicherheit (drug safety) oder aber zur Erforschung von Grundlagen von Krankheiten und physiologischen Zusammenhängen in den Organsystemen verwendbar sind.

Nachteilig ist, dass derzeit keine Gefäßversorgung bei der Kultivierung solcher Organoide erfolgt und besonders auch die Entwicklung der Organoide über einen bestimmten embryonalen Reifegrad hinaus aber auch die Interaktion mit nicht im Organoid enthaltenen Zelltypen oder bei deren unphysiologischer Zellorientierung nicht gewährleistet werden kann. So kommt es in bekannten Kultivierungsverfahren vermutlich aufgrund fehlender Versorgung und Interaktion zu Mangelversorgung, Zelluntergang und zu unphysiologischen Zuständen, was die Verwertbarkeit der in vitro gefundenen Erkenntnisse erschwert.

Ein besonders vielversprechendes Organoidsystem sind sogenannte Retina-Organoide, welche die komplexen Interaktionen in der mehrschichtigen Retina simulierbar machen sollen. Retina-Organoide können aus insbesondere patientenspezifischen iPS-Zellen gewonnen werden und beinhalten alle Zelltypen der Neuralen Retina: Photorezeptoren, retinale Neurone und Gliazellen, in einem komplexen Zusammenspiel ähnlich einer embryonalen Situation. Dabei sind die Prophylaxe und Therapie häufiger und schwerer Erkrankungen der Retina, wie die altersbedingte Makuladegeneration (AMD) oder die Retinitis Pigmentosa (RP), welche Hauptursachen für Erblindungen beim Menschen sind, eine wichtige Motivation zur Entwicklung möglichst physiologischer Retina-Organsysteme. Für das Modellieren einer möglichst physiologischen Retina fehlt den derzeitigen Retina-Organoid-Systemen allerdings a) die interzelluläre Interaktion der Photorezeptoren (vor allem von deren Außensegmente) mit retinalen Pigmentepithelzellen (RPE), b) die Einbindung von subpigmentepithelialen Endothelzellen und Gefäßen der Choroidea und c) eine physiologische Extrazellulärmatrix (EZM), vor allem im Interaktionsbereich der RPE, die so genannte Interphotorezeptormatrix.

Der vorliegenden Erfindung lag das technische Problem zugrunde, Verfahren und Mittel zur verbesserten Kultivierung von organähnlichen Organsystemen zur Erforschung und Entwicklung prophylaktischer oder therapeutischer Mittel aus insbesondere patientenspezifischen Zellen, vornehmlich iPS-Zellen, bereitzustellen, welche die genannten Nachteile bekannter Organoidkulturen, insbesondere unvollständige Reifung, Zelluntergang und fehlende Zellinteraktion, überwinden.

Das technische Problem wird vollständig gelöst durch ein Verfahren zur Co-Kultivierung von Organoid-Gewebe in einem Bioreaktorgefäß mit bodenseitiger semipermeabler Membran, in insbesondere mikrophysiologischem Maßstab. Das Verfahren enthält zumindest die Schritte: Schritt (a): Aussäen von Zellen mindestens eines ersten Zelltyps in das Bioreaktorgefäß auf die Membran, Schritt (b): Kultivieren dieser ausgesäten Zellen, so dass sich an der Membran eine, insbesondere konfluente, geträgerte 2D-Zellschicht ausbildet, und insbesondere unmittelbar anschließend Schritt (c): Einbringen des Organoids, enthaltend Zellen mindestens zweier weiterer Zelltypen, die in definierter 3D-Struktur zueinander angeordnet sind, und von- Hydrogel in das Bioreaktorgefäß (30) und auf die geträgerte 2D-Zellschicht (20), und zwar mit der Maßgabe, dass der Organoid in dem Bioreaktorgefäß, in das er eingebracht wird durch das ebenfalls, zuvor und/oder gleichzeitig, eingebrachte Hydrogel von der geträgerten 2D-Zellschicht auf einem definierten Abstand gehalten wird. Dieser Abstand entspricht bevorzugt den Dimensionen zwischen den interagierenden Zellen in vivo; dies wird unten näher erläutert.

Die Erfindung sieht also besonders vor, einen 3D-Organoid mit 3D-Strukur mehrerer Zelltypen über mindestens einer 2D-Zellschicht, das heißt besonders einem Monolayer, aus Zellen mindestens eines weiteren Zelltyps in definiertem Abstand zu positionieren. Dadurch wird eine kontrollierbare physiologisch adäquate Interaktionen zwischen mindestens einem Zelltyp des 3D-Organoids und dem mindestens einen weiteren Zelltyp des darunter liegenden 2D-Monolayers ermöglicht. Eine physiologisch adäquate Versorgung der Zellen des 3D-Organoids über den darunter liegenden 2D-Monolayer wird gegebenenfalls auch ermöglicht. Dadurch gelingt es, den bereitgestellten Organoid unter physiologisch adäquaten Bedingungen zu kultivieren; eine verbesserte Reifung und Ausdifferenzierung des Organgewebes wird erreicht und unerwünschter Zelltod wird vermieden. Vorteilhafterweise kann eine stabile Interaktion zwischen dem 3D-Organoid und der 2D-Zellschicht erreicht und folglich untersucht werden, so dass eine organtypische Sandwichkultur, welche die komplexe Struktur und Funktion von in vivo Organen widerspiegelt, erhalten werden.

In einer bevorzugten Variante ist vorgesehen, dass in Schritt (c) die weitere Maßgabe gilt, dass durch das eingebrachte Hydrogel der Organoid in dem Bioreaktorgefäß zusätzlich auch von den Wänden des Bioreaktorgefäßes beabstandet wird. Gemäß dieser bevorzugten Variante schwebt der eingebrachte Organoid gleichsam in dem Bioreaktorgefäß ohne mechanischem Kontakt zu den Gefäßwänden und in definiertem, das heißt kontrollierbarem oder vorbestimmbarem, Abstand zu der mindestens einen darunterliegenden 2D-Zellschicht. Dies erlaubt eine weitere Verbesserung der Ergebnisse der Kultivierung des Organoids: Es tritt weniger Zelltod auf, und die insbesondere selbstorganisierende Strukturierung der Zelltypen innerhalb des Organoids setzt sich im Laufe der Kultivierung fort in Richtung einer adulten, das heißt weitgehend vollständig ausgebildeten Schichtung und Struktur der enthaltenen Zellen.

Um die erfindungsgemäße Beabstandung des 3D-Organoids von der 2D-Zellschicht in dem Bioreaktorgefäß zur ermöglichen ist in einer ersten Variante vorgesehen, dass in Schritt (c) der Organoid zusammen mit dem Hydrogel in das Bioreaktorgefäß eingebracht wird. Dabei ist besonders vorgesehen, dass der Organoid in einem definieren Volumen an Hydrogel, das zunächst in flüssiger Phase vorliegt, in das Gefäß und auf die geträgerte 2D-Zellschicht gegossen oder gegebenenfalls gepumpt wird und das Hydrogel dort aushärtet, während der Organoid in dem Hydrogel schwimmt und dabei in definiertem Abstand über der 2D-Zellschicht schwebt und gegebenenfalls auch von den Wänden des Gefäßes beabstandet ist. Der gewünschte definierte Abstand wird dabei besonders durch den Zeitverlauf des Übergangs (Aushärten) von flüssiger zur festen Phase des in Schritt (c) eingegebenen Hydrogels in Verbindung mit dessen Viskosität bestimmt. Die Viskosität bestimmt die Zeit des Absinkens des Organoids in dem Hydrogel in Richtung der darunterliegenden 2D-Zellschicht. Härtet das Hydrogel aus, wird ein weiteres Absinken des Organoids verhindert und ein vorbestimmbarer Abstand zu der 2D-Zellschicht erreicht.

In einer alternativen Variante ist vorgesehen dass Schritt (c) Teilschritte enthält, und zwar zumindest einen ersten Teilschritt (c1): Einbringen eines Teils des Hydrogels, bevorzugt als flüssiges Sol und damit verbunden ein Aushärten zu einem Gel, um zunächst eine definierte Abstandschicht aus Hydrogel zu der 2D-Zellschicht zu bilden, und anschließend einen zweiten Teilschritt (c2): Einbringen des Organoids auf die gebildete Hydrogel-Abstandsschicht, und zwar bevorzugt in Hydrogel als flüssigem Sol und damit verbunden ein Aushärten zu einem Gel, um den Organoid in dem Gefäß, gegebenenfalls auch von den Wänden des Gefäßes beabstandet, zu fixieren. Der gewünschte definierte Abstand wird dabei besonders durch die Menge/das Volumen des in Schritt (c1) eingegebenen Hydrogelanteils bestimmt.

In einer bevorzugten Ausgestaltung ist außerdem vorgesehen, dass bei der Kultivierung die mindestens eine an der Membran geträgerte 2D-Zellschicht an ihrem basalen Pol, das heißt die von dem Organoid abgewandte Seite, getrennt von ihrem apikalen Pol, das heißt die zu dem Organoid gewandte Seite, perfundiert wird. Dadurch wird eine adäquate physiologische Versorgung des kultivierten Organoids durch die mindestens eine 2D-Zellschicht hindurch erreicht. Weiter kann eine adäquat physiologische Funktion der mindestens einen 2D-Zellschicht, vor allem wenn diese eine Polarisierung in apikalen und basalen Pol ausbildet oder ausbilden soll, erreicht werden. Damit vorteilhafterweise verbunden ist eine adäquat physiologische Interaktion der 2D-Zellschicht mit dem Organoiden. So kann ein optimal verbessertes in vitro Organoid-System bereitstellt werden, das weitgehend die physiologischen Zustände und Funktionen des Organs in vivo nachbildet.

Gegenstand der Erfindung ist demgemäß auch eine in vitro Gewebekultur, die mittels des erfindungsgemäßen Verfahrens herstellbar ist. Diese in vitro Gewebekultur ist erfindungsgemäß in einem Bioreaktorgefäß mit bodenseitiger semipermeabler Membran enthalten und enthält oder besteht bevorzugt aus: mindestens einer 2D-Zellschicht, enthaltend mindestens einen ersten Zelltyp, geträgert an der semipermeablen Membran, einem Organoiden, enthaltend Zellen mindestens zweier weiterer Zelltypen, die in definierter 3D-Struktur zueinander angeordnet sind, und Hydrogel, worin der Organoid in dem Bioreaktorgefäß eingebettet und zu der geträgerten 2D-Zellschicht in einem definierten Abstand angeordnet und bevorzugt auch zu den Wänden des Bioreaktorgefäßes beabstandet ist.

In bevorzugten Varianten beträgt der definierte Abstand des Organoids zu der 2D-Zellschicht von 1 bis 200 µm, bevorzugt von 1 bis 100µm, mehr bevorzugt von 2 bis 20 µm, besonders bevorzugt 2 bis 15µm beträgt.

Alle Zellen können humanen oder tierischen Ursprungs sein; bevorzugt sind Maus und Ratte, Die Zellen können aus embryonalen oder bevorzugt alternativ aus induziert pluripotenten Stammzellen (iPS) gewonnen werden. Sie können aus embryonalem oder adultem Gewebe entnommen worden sein; menschliche embryonale Zellen sind ausgeschlossen.

In einer besonderen Ausgestaltung der in vitro Gewebekultur ist der mindestens eine erste Zelltyp der 2D-Zellschicht ausgewählt aus: Epithelzellen, Pigenteptihelzellen oder epithelartige Zelllinien, wie ARPE-19; Endothelzellen; Stromazellen, enthaltend Fibrozyten und/oder Fibroblasten; und Muskelzellen, enthaltend Myoblasten, Myozyten und/oder Muskelfasern. Besonders bevorzugt sind Epithelzellen, bevorzugt in Kombination mit Endothelzellen, wobei eine erste 2D-Zellschicht, enthaltend und bevorzugt bestehend aus Epithelzellen, auf der Oberseite der Membran die dem Organoid zugewandt ist, angeordnet ist. Besonders bevorzugt ist dabei eine weitere 2D-Zellschicht auf der Unterseite der Membran, die dem Organoid abgewandt ist, angeordnet, welche Endothelzellen enthält und bevorzugt daraus besteht. In einer bevorzugten Variante sind als Zelltypen der 2D-Zellschichten menschliche embryonale Zellen, insbesondere embryonale Stammzellen als solche, ausgenommen, die 2D-Zellschichten damit frei von solchen Zellen.

Bevorzugt ist der Organoid ausgewählt ist aus der Gruppe der selbstorganisierenden oder mittels Zelldruck herstellbaren Mehrzelltyp-Geweben mit definierter 3D-Struktur. Diese Gruppe enthält bevorzugt Retina-Organoide, Gehirn-Organoide, Pankreas-Organoide, und Darm-Organoide oder besteht vorzugsweise ausschließlich daraus. In einer bevorzugten Variante sind als Zellen des 3D-Organoids menschliche embryonale Zellen, insbesondere embryonale Stammzellen als solche, ausgenommen, der Organoid damit frei von solchen Zellen.

In einer besonders bevorzugten Ausführung der in vitro Gewebekultur ist der Organoid ein Retina-Organoid, der zumindest Photorezeptorzellen und Zellen mindestens eines weiteren Zelltyps der neuralen Wirbeltier-Retina enthält. In dieser Ausführung ist die geträgerte 2D-Zellschicht eine konfluente Monolage aus retinalen Pigmentepithelzellen. Bevorzugt ist eine weitere 2D-Zellschicht auf der Unterseite der Membran, die dem Organoid abgewandt ist, angeordnet, welche aus Endothelzellen besteht.

Die Erfindung betrifft auch die Verwendung des erfindungsgemäßen Co-Kultivierungsverfahrens und der erfindungsgemäßen in vitro Gewebekultur zur Entwicklung und/oder Auswahl prophylaktischer und/oder therapeutischer Mittel und Wirkstoffe insbesondere in patientenspezifischen prophylaktischen und/oder therapeutischen Verfahren, besonders in der individualisierten Therapie. Bevorzugt sieht dieses Verfahren vor, den 3D-Organoid und besonders auch die mindestens eine 2D-Zellschicht aus isolierten Zellen des Patienten. besonders aus iPS-Zellen zu gewinnen.

Die Erfindung wird durch die Figuren und nachfolgenden Beispiele erläutert:

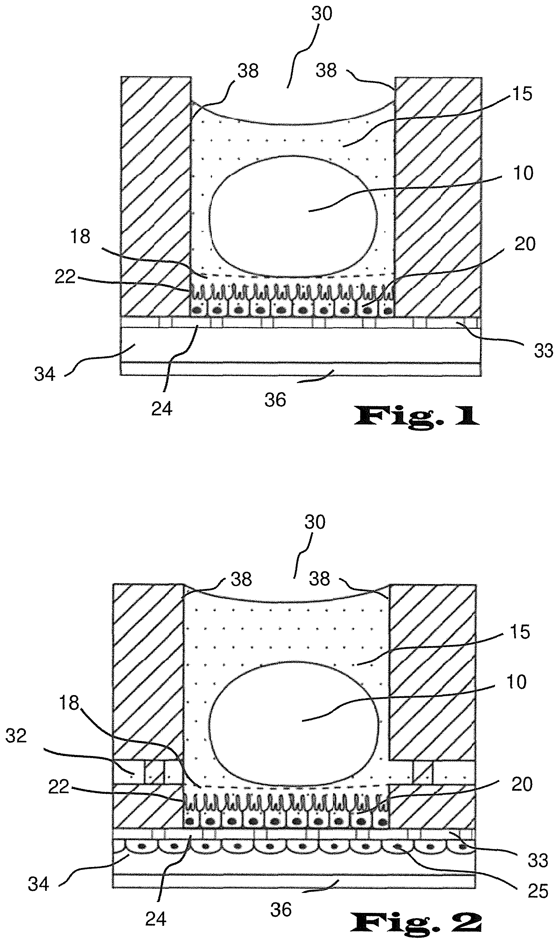

Figur 1 zeigt eine schematische Schnittansicht einer ersten Ausführung des Bioreaktorsystems mit einem Bioreaktorgefäß (30) auf einem Träger (36) mit bodenseitiger semipermeabler Membran (33). Darin angeordnet ist die erfindungsgemäß kultivierte in vitro Gewebekultur, bestehend aus einem 3D-Organoid (10), der co-kultiviert ist mit mindestens einer in definiertem Abstand dazu angeordneten, bevorzugt einlagigen 2D-Zellschicht (20), die auf der Membran (33) geträgert ist. Der Organoid (10) in dem Bioreaktorgefäß (30) schwebt gleichsam über der darunterliegenden 2D-Zellschicht (20). In der dargestellten Ausführung ist die 2D-Zellschicht (20) eine konfluente Zelllage aus Epithelzellen. Der apikale Pol (22) der Zelllage weist Richtung Organoid, der basale Pol (24) weist in die entgegengesetzte Richtung; er liegt in der dargestellten Ausführung auf der Membran (33) auf. In dem Bioreaktorsystem ist ein basaler Perfusionskanal (34) vorgesehen, der eine Perfusion des basalen Pols (24) der 2D-Zellschicht (20) erlaubt. Der Träger (36) ist bevorzugt ein durchsichtiges Substrat, ein Glasobjektträger um die Durchlichtmikroskopische Untersuchung zu ermöglichen.
Figur 2 zeigt eine schematische Schnittansicht einer Variante der Ausführung des Bioreaktorsystems nach Figur 1. Die semipermeable Membran (33) weist auf ihrer zu dem Organoid gewandten Seite eine erste 2D-Zellschicht (20) und auf der gegenüberliegenden, basalen Seite eine zweite 2D-Zellschicht (25) auf. In der dargestellten Ausführung ist die erste 2D-Zellschicht (20) eine konfluente Zelllage aus Epithelzellen; die zweite 2D-Zellschicht (25) eine konfluente Zelllage aus Endothelzellen. Über den basalen Perfusionskanal (34) ist eine Perfusion des basalen Pols (24) der 2D-Zellschicht (20) ermöglicht. Ein zusätzlicher apikaler Perfusionskanal (32) erlaubt die getrennte Perfusion der apikalen Seite der 2D-Zellschicht (20) und des Organoids (10).

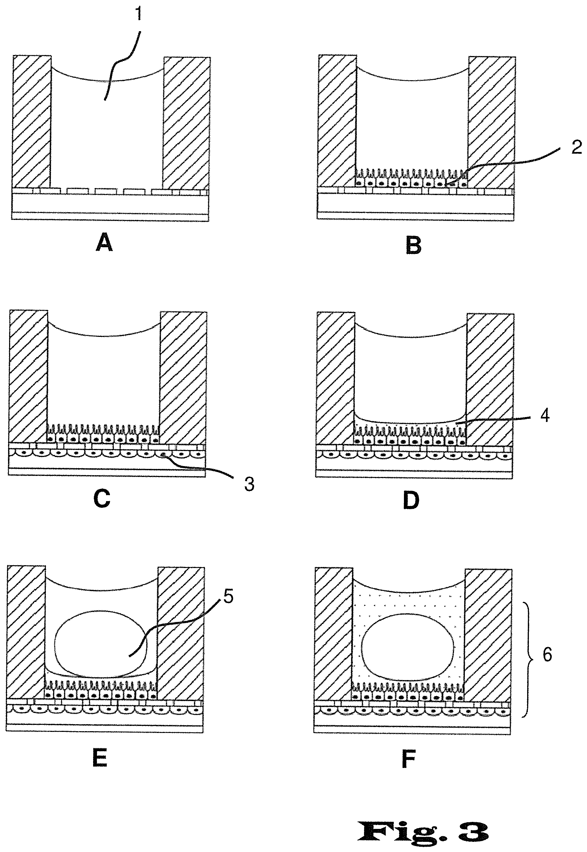

Figur 3 zeigt, in Einklang mit den schematischen Darstellungen in den Figuren 1 und 2, die Phasen der Besiedelung Bioreaktorsystems und Kultivierung des gebildeten in vitro Gewebes anhand eines Retina-Organoids (RO) in Co-Kultur mit retinalem Pigmentepithel (RPE) und Endothelschicht (EN): In Schritt A wird ein Bioreaktorgefäß mit bodenseitiger semipermeabler Membran bereitgestellt. Die Membran wird optional durch Inkubation mit Laminin (1) beschichtet. In Schritt B werden einzelne RPE-Zellen (2) in Suspension auf die Membran gegeben und inkubiert, bis diese zu Konfluenz wachsen. In Schritt C wird die basale Seite der Membran mit Endothelzellen besiedelt. In Schritt D wird auf die RPE-Zelllage (2) eine erste Schicht Hydrogel (4) gegeben, die als Abstandslage dient. In Schritt E wird der Retina-Organoid (5) zusammen mit Hydrogel auf die Abstandslage (4) verbracht. Es bildet sich in Schritt F die finale in vitro Gewebekultur (6), bestehend aus RO, eingebettet in Hydrogel und definiert beabstandet zu RPE und basalem Endothel. Ab Schritt F findet die physiologische Ausreifung des RO und die physiologische Interaktion von Photorezeptoren des RO mit den RPE-Zellen statt. Eine Perfusion des Retina-Organoids erfolgt in der dargestellten Ausführung über den basalen Perfusionskanal des Bioreaktors durch die Endothel- und Pigmentepithelschicht hindurch statt.

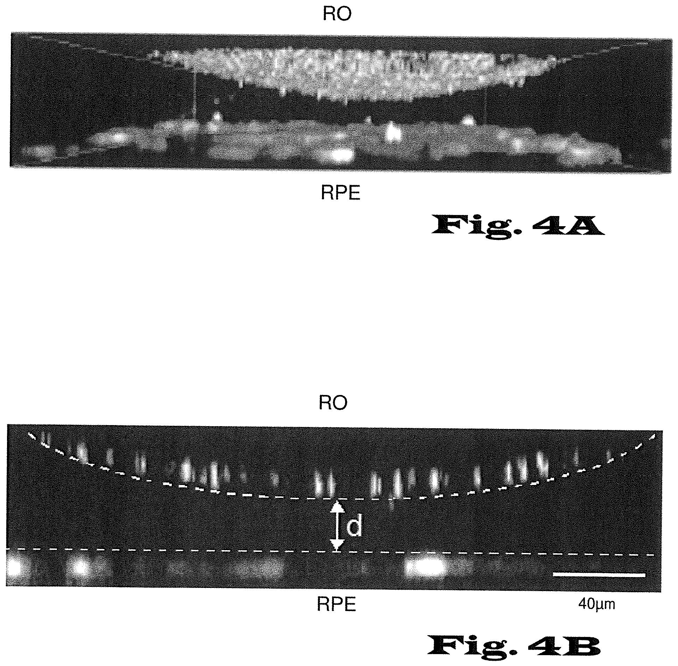

Die Figuren 4A und B zeigen "live cell imaging" mit Fluorophor-markiertem iPS-RPE und iPS-RO, deren Außensegmente mit Fluorophor-gekoppeltem PNA-Lectin (Erdnuss-Agglutinin) markiert wurden (Figur 4A). Die definierte Positionierung des iPS-RPE Gewebes zum iPS-RO ist in Figur 4B gezeigt: Markierung von inneren/äußeren Segmenten des iPS-RO mittels PNA-Lectin mit anschließender Co-Kultivierung mit GFP markierten iPS-RPE; die Grenzen des RPE (unten) sowie des sphäroiden RO (oben) sind gestrichelt eingezeichnet (Maßstab: 40 µm, d= Abstand RO-RPE).

### Beispiel: Mikrophysiologisches System der menschlichen Retina (MPS)

### 1. Herstellung/Zusammenbau des Retina MPS

Zur Herstellung des Bioreaktors werden Schichten des MPS werden mittels Abformung von Polydimethylsiloxan (PDMS) an mikrostrukturierten Siliziumwafern hergestellt. Die Herstellung des MPS ist aber nicht auf dieses Material limitiert, und weitere Materialen wie Glas, PC sowie PET und deren Kombination sind möglich. Die Mikrostrukturierung der jeweiligen Abgussformen (Master), wird durch UV-Lithographie von Fotolack (SU-8; Firma MicroChem) realisiert.

Die Abformung der Endothel/Mediumsschicht erfolgt mittels so genanntem "exclusion molding" (EM). In der Variante des MPS mit einer zusätzlichen Medienversorgung des RO, wird ebenfalls die RPE-Gewebeschicht mittels EM hergestellt.

Die semipermeable Membran aus Materialen wie z.B. PET sollte eine Porengröße von 2 µm - 3 µm sowie eine Dicke von 10 µm - 30 µm aufweisen. Für die irreversible Verbindung von PDMS und PET findet eine Funktionalisierung der Membran mit bis-Aminosilan aus der flüssigen Phase auf die vorher mit Sauerstoffplasma behandelte Membran statt. Durch diese zusätzliche Beschichtung entsteht eine irreversible Verbindung von Membran zu den später ebenfalls Sauerstoffplasma behandelten PDMS Schichten.

Der Zusammenbau des MPS findet in mehreren Schritten statt. Zuerst wird auf einen Objektträger Glas mit einer Stärke von 0,17 mm - 1 mm die abgeformte Endothel/basale Perfusion-Schicht auf einer Trägerfolie nach Aktivierung im Sauerstoffplasma gebracht und angedrückt. Um die Verbindung zu verstärken, werden diese in einem Konvektionsofen bei 60 °C - 80 °C erhitzt. Die Trägerfolie wird von der Endothel/basale Perfusion-Schicht abgezogen. Die Aufbringung der semipermeablen Membran sowie der RO- & RPE-Gewebeschicht erfolgt in mehreren Schritten. Hierzu werden vorab die Durchgangslöcher für die Zu- und Abläufe in den darunter liegenden Schichten geschaffen. Die funktionalisierte semipermeable Membran wird in die dafür vorgesehene Einlagefläche eingelegt. Als letzter Schritt, wird die RO- & RPE-Gewebeschicht mit der Membran auf die Endothel/basale Perfusion-Schicht aufgelegt und angedrückt und für die Dauer von 10 Stunden - 24 Stunden in einem Konvektionsofen auf 60 °C bis 80 °C erhitzt.

Mehrere solcher Bioreaktorgefäße können in einem MPS auf einem gemeinsamen Träger nebeneinander angeordnet sein.

### 2. Etablierung der Co-Kultivierung in MPS

Zu Beginn wird das assemblierte MPS mittels eines Sauerstoffplasmas bei einer Leistung von 50 Watt und einem Sauerstoffgasfluss von 0,1 Nml/min - 0,3 Nml/min sowie einer Behandlungsdauer von 5 bis 15 Minuten sterilisiert. Eine Sterilisierung des MPS kann ebenfalls mittels Autoklavieren oder Gammabestrahlung erfolgen. Nach Durchführung der Plasmabehandlung erfolgt eine Beschichtung der semipermeablen Membran, um ein späteres Adhärieren der RPE Zellen zu ermöglichen. Hierzu wird eine 1:10 - 1:25 Verdünnung von DMEM/F12 und Laminin aufgebracht und das MPS für eine Dauer von 1 bis 4 Stunden bei 37 °C und 5% CO2 inkubiert (Figur 3A). Vor der Ausplattierung der iPS-RPE Zellen wird das überschüssige Laminingemisch entfernt und das komplette MPS mit Medium gespült. Die bereits vorher vereinzelte iPS-RPE Zellen werden in einem Volumen von 5 µl - 10 µl direkt von oben durch die RO- & RPE-Gewebekammer auf die Membran gegeben. iPS RPE Zellen werden für eine Dauer von 30 bis 60 Minuten bei 37°C und 5% CO2 inkubiert um ein Adhärieren der iPS RPE Zellen auf der Membran zu ermöglichen (Figur 3B). Anschließend wurden die iPS RPE Zellen im MPS für eine Dauer von 1 bis 3 Tagen extern über Spritzenpumpen bei einem konstanten Mediumfluss von 10 µl/Stunde - 20 µl/Stunde kultiviert.

Nachdem die iPS RPE Zellen die Membran konfluent bewachsen haben, erfolgt die Einbringung der iPS Endothelzellen auf der Unterseite der Membran (Figur 3C). Hierzu wurden vorher vereinzelte iPS Endothelzellen in den Endothel/Medienkanal injiziert und das MPS auf dem Kopf gestellt damit diese auf die Membran Unterseite absinken. Um ein vollständiges Adhärieren der iPS Endothelzellen auf der Membran zu erlauben wurde das MPS für eine Dauer von 30 bis 60 Minuten bei 37 °C und 5% CO2 inkubiert. Anschließend wurde das MPS wieder extern über eine Spritzenpumpe bei einer Flussrate von 10 µl/Stunde - 20 µl/Stunde versorgt. Nachdem die iPS Endothelzellen die Unterseite der Membran konfluent bewachsen haben, wurde für die präzise Distanzierung von iPS-RPE Zellen zu den einzelnen iPS-RO ein Hydrogel (im speziellen eines Hyaluron-basierten Hydrogel) eingebracht (Figur 3D). Das Hydrogel wurde direkt über den RO- & RPE-Gewebekanal oder durch obere Öffnung der jeweiligen RO- & RPE-Gewebekammer injiziert, um eine späteren Abstand zwischen iPS-RPE und iPS-RO von 10 µm - 50 µm zu realisieren.

Nach vollständiger Verfestigung des Hydrogels, wurde jeweils ein iPS-RO je Bioreaktorgefäß direkt von oben appliziert (Figur 3E). Um eine Bewegung der iPS-ROs über die Dauer der Kultivierung zu verhindern, wurden diese mittels dieses Hydrogels fixiert. Die Einbringung es Hydrogels findet durch die direkte Zugabe durch die Oberseite der RO- & RPE-Gewebekammer statt.

Anschließend wurde die externe Mediumversorgung des MPS bei einer Flussrate von 10 µl/Stunde - 20 µl/Stunde wieder hergestellt. Das MPS wurde im Anschluss für eine Dauer von 1 bis 7 Tagen kultiviert, um eine Interaktion zwischen RPE und Photorezeptoren der ROs zu verfolgen und analysieren, sowie den Einfluss verschiedener Wirkstoffe zu untersuchen (Figur 3F).

### 3. Anwendung des Retina MPS

Die physiologische Funktionalität und Vitalität die Co-Kultivierung der iPS-RO und iPS-RPE im MPS war wie folgt nachweisbar:
3.1 Vitalitätsnachweis durch "live cell imaging" mit Hilfe von Fluophormarkiertem iPS-RPE und iPS-RO, deren Außensegmente mit Flurophorgekoppeltem PNA-Lektin (Erdnuss-Agglutinin) markiert wurden (Figuren 4A und 4B). Eine optische Rekonstruktion durch konfokale Mikroskopie konnte eine Annäherung sowie Interaktion beider Gewebe im MPS zeigen. Vitalität der verschiedenen Gewebe für eine Kultivierungsdauer von bis zu 7 Tagen.
3.2 Nachweis zur Erhaltung der Gewebestruktur und Beurteilung der Morphologie durch Lichtmikroskopie und Elektronenmikroskopie im MPS: Beide Gewebe wiesen hierbei das erwartete Erscheinungsbild auf und es konnten keine Anzeichen für Apoptose oder Aktivierung von Gliazellen gefunden werden. Zudem konnte auf der Organoidseite die dem RPE gegenüberliegt eine Ausbildung von großen, Außensegment-ähnlichen Strukturen beobachtet werden (mit Hilfe von Peripherin2 und Rhodopsin als Marker). Diese Strukturen konnten auf der RPEabgewandten Seite des Organoids, sowie unter konventioneller Organoidkultur nicht nachgewiesen werden.
3.3 Immunohistologischer Nachweis von typischen retinalen Biomarkern: Für die Immunhistologie der iPS-RO, wurden diese im Anschluss an die Kultur aus dem MPS isoliert und Schnitte angefertigt. Die Immunhistologie der iPS-RPE Zellen erfolgte direkt im MPS. Die Expression der wichtigsten Biomarker von sowohl iPS-RPE Zellen als auch iPS-RO (ZO-1, MiTF, RPE65, CHX10, ARR3, RHOD) konnte gezeigt werden.
3.4 Real-Time quantitative PCR (qPCR) zum Nachweis der Expression typischer retinaler Biomarker: Es wurden hierzu iPS-RO nach einer Dauer von 3 Tagen welche einerseits mit iPS-RPE Zellen oder ohne iPS-RPE Zellen im MPS co-kultiviert worden. Diese wurden mit konventionellen kultivierten iPS-RO anschließend verglichen. Es konnten für alle retinalen Zelltypen eine vergleichbare Expression der entsprechenden Marker auf mRNA Ebene nachgewiesen werden. Ebenso wurde die iPS-RPE-Kultur im MPS mit der konventionellen Kultur verglichen und es konnten auch keine signifikanten Unterschiede in der Expression verschiedener RPE-Marker festgestellt werden.
3.5 Nachweis der Funktionalität der Co-Kultivierung von iPS-RO und iPS-RPE im MPS mittels Calcium-Imaging und Phagozytose-Assay: Es konnten spontane Calciumströme innerhalb der Photorezeptorzellen nachgewiesen werden, was auf ein physiologisches Verhalten der Photorezeptorene auch im MPS hindeutet. Weiterhin lässt sich durch die PNA-Lektin Markierung der Außensegmente auch deren physiologische Abstoßung durch die Photorezeptorzelle beobachten. Das iPS-RPE wies nicht nur eine Aufnahme und Phagozytose von extern applizierten, bovinen Außensegmenten im sog. Phagozytose-Assay auf, sondern zeigte in der Live cell Mikroskopie auch die Aufnahme eben dieser PNA-Lektin markierten Außensegmente im MPS.
3.6 Etablierung von zusätzlichen in-situ Messverfahren im MPS: CLARITY, eine Methode zur optischen Klärung von Gewebeproben wurde verwendet um eine ganzheitliche immunhistologische Färbung direkt im MPS zu ermöglichen. Auch durch diese Methode konnten typische retinale Biomarker nachgewiesen werden. Eine weitere Methode um iPS-RO im MPS unter Echtzeitbedingungen zu beobachten, stellt die Verwendung von Reporterzellinien dar welche unter dem Promoter des jeweiligen aktivierten Markergens für den entsprechenden Zelltyp eine Flurophorexpression aufweisen und somit ein Live cell Imaging innerhalb des MPS ermöglichen. Es liesen sich durch diese Methode Photorezeptoren, deren Außensegmente, retinale Ganglienzellen, sowie aktivierte Gliazellen unter Echtzeitbedingungen nachweisen.
3.7 Verwendung des MPS in der medizinischen Wirkstofftestung: Hierfür wurden Medikamente verwendet mit bekannten retinopathischen unerwünschten Arzneimittelwirkungen. Das Antiepileptikum Vigabtratin (VB), ein GABA Transaminase Inhibitor, zählt zu diesen Stoffen. Die iPS-RO im MPS wurden hierzu für eine Dauer von 20 Tage mit VB behandelt. Zwar ließ sich keine morphologische Veränderung oder Aktivierung der Gliazellen im iPS-RO nachweisen. Jedoch konnte durch eine zusätzliche Lichtexposition des iPS-RO, eine erhöhte Lichtaktivität nach 11 Tagen VB-Behandlung festgestellt werden. Außerdem führte bereits eine akute VB-Behandlung unter Licht zu vermehrten spontanen Calciumströmen. Dieser elektrophysiologischer Effekt durch die Behandlung mit VB im MPS, lieferte somit einen Nachweis der retinopathischen Nebenwirkungen die auch im Patienten beobachtet werden. Des Weiteren wurden die Effekt von Chloroquin, einem Malariamittel mit ebenfalls bekannten retinopathischen Wirkungen untersucht, welches zu einer sog. Chloroquinretinopathie führt. Versuche mit iPS-RPE Zellen unter konventionellen Kultivierungsbedingungen, sowie im MPS wiesen eine Vaskularisierung auf, die auf eine pathologische Vergrößerung der Lysosomen zurückzuführen ist. Dies konnte durch Immunohistologische Färbung von Lamp2 als lysosomalen Marker gezeigt werden. Schließlich wurde auch die Wirkung des Chloroquins auf den iPS-RO im MPS untersucht. Nach 2-tägiger Behandlung der iPS-RO, konnte eine Aktivierung der Gliazellen beobachtet werden, welche mit Hilfe eines GFAP-Promoter Konstruktes markiert wurden.

## Patentansprüche

1. Verfahren zur mikrophysiologischen Co-Kultivierung von Organoid-Gewebe (10) in einem Bioreaktorgefäß (30) mit bodenseitiger semipermeabler Membran (33), enthaltend die Schritte:
(a) Aussäen von Zellen mindestens eines ersten Zelltyps auf die Membran (33),
(b) Kultivieren dieser ausgesäten Zellen zur Ausbildung mindestens einer an dieser Membran (33) geträgerten 2D-Zellschicht (20) und
(c) Einbringen in das Bioreaktorgefäß (30) auf die geträgerte 2D-Zellschicht (20) von
- Organoid (10), enthaltend Zellen mindestens zweier weiterer Zelltypen, die in definierter 3D-Struktur zueinander angeordnet sind, und
- Hydrogel (15)
mit der Maßgabe, dass der Organoid (10) in dem Bioreaktorgefäß (30) durch das eingebrachte Hydrogel (15) von der geträgerten 2D-Zellschicht (20) definiert beabstandet wird.

2. Verfahren nach Anspruch 1, wobei in Schritt (c) die weitere Maßgabe gilt, dass der Organoid (10) in dem Bioreaktorgefäß (30) durch das eingebrachte Hydrogel (15) zusätzlich auch von den Wänden (38) des Bioreaktorgefäßes (30) beabstandet wird.

3. Verfahren nach Anspruch 1 oder 2, wobei in Schritt (c) der Organoid (10) zusammen mit dem Hydrogel (15) in das Bioreaktorgefäß (30) eingebracht wird.

4. Verfahren nach Anspruch 1 oder 2, wobei Schritt (c) die Teilschritte enthält:
(c1) Einbringen eines Teils des Hydrogels, um eine definierte Abstandschicht (18) zu der 2D-Zellschicht (20) und gegebenenfalls zu den Wänden (38) des Bioreaktorgefäßes (30) zu bilden, und anschließend
(c2) Einbringen des Organoids (10) auf die gebildete Hydrogel-Abstandsschicht (18).

5. Verfahren nach einem der vorstehenden Ansprüche, wobei bei der Kultivierung der 2D-Zellschicht (20) an der bodenseitigen semipermeablen Membran (33) an ihrem basalen Pol (24) getrennt von ihrem apikalen Pol (22) perfundiert wird.

6. In vitro-Gewebekultur in einem Bioreaktorgefäß (30) mit bodenseitiger semipermeabler Membran (33), enthaltend:
- 2D-Zellschicht (20) enthaltend mindestens einen ersten Zelltyp an der semipermeablen Membran (33),
- Organoid (10), enthaltend Zellen mindestens zweier weiterer Zelltypen, die in definierter 3D-Struktur zueinander angeordnet sind, und
- Hydrogel (15), worin der Organoid (10) in dem Bioreaktorgefäß (30) eingebettet und zu der bodenseitigen 2D-Zellschicht (20) über einen definierten Abstand beabstandet ist.

7. In vitro-Gewebekultur nach Anspruch 6, wobei der definierte Abstand des Organoids (10) zu der 2D-Zellschicht (20) 1 bis 100 µm, bevorzugt 2 bis 20 µm beträgt.

8. In vitro-Gewebekultur nach Anspruch 6 oder 7, wobei der Organoid (10) in dem Hydrogel (15) so eingebettet ist, dass er außerdem zu den Wänden (38) des Bioreaktorgefäßes (30) beabstandet ist.

9. In vitro-Gewebekultur nach einem der Ansprüche 6 bis 8, wobei eine erste 2D-Zellschicht (20) auf der Oberseite der Membran (33), die dem Organoid (10) zugewandt ist, angeordnet ist.

10. In vitro-Gewebekultur nach Anspruch 9, wobei eine weitere 2D-Zellschicht (25) auf der Unterseite der Membran (33), die dem Organoid (10) abgewandt ist, angeordnet ist.

11. In vitro-Gewebekultur nach einem der Ansprüche 6 bis 10, wobei der Zelltyp der 2D-Zellschicht (20,25) ausgewählt ist aus:
- Epithelzellen,
- epithelartige Zellen,
- Endothelzellen,
- Stromazellen, enthaltend Fibrozyten und/oder Fibroblasten, und
- Muskelzellen, enthaltend Myoblasten, Myozyten und/oder Muskelfasern.

12. In vitro-Gewebekultur nach einem der Ansprüche 9 bis 11, wobei die erste 2D-Zellschicht (20) auf der Oberseite der Membran (33), die dem Organoid (10) zugewandt ist, Epithelzellen enthält oder daraus besteht.

13. In vitro-Gewebekultur nach Anspruch 12, wobei die weitere 2D-Zellschicht (25) auf der Unterseite der Membran (33), die dem Organoid (10) abgewandt ist, Endothelzellen enthält oder daraus besteht.

14. In vitro-Gewebekultur nach einem der Ansprüche 6 bis 13, wobei der Organoid (10) ausgewählt ist aus der Gruppe der selbstorganisierenden oder mittels Zelldruck herstellbaren Mehrzelltyp-Geweben mit definierter 3D-Struktur, enthaltend: Retina-Organoide, Gehirn-Organoide, Pankreas-Organoide, und Darm-Organoide.

15. In vitro-Gewebekultur nach einem der Ansprüche 6 bis 14, wobei der Organoid (10) ein Retina-Organoid ist, der zumindest Photorezeptorzellen und Zellen mindestens eines weiteren Zelltyps der neuralen Wirbeltier-Retina enthält, und die 2D-Zellschicht (20) eine konfluente Monolage aus retinalen Pigmentepithelzellen ist.

## Claims

1. A method for the microphysiological co-cultivation of organoid tissue (10) in a bioreactor vessel (30) with a semi-permeable membrane (33) on the bottom, comprising the steps:
(a) seeding cells of at least one first cell type onto the membrane (33),
(b) cultivating these seeded cells to form at least one 2D cell layer (20) supported on this membrane (33) and
(c) introducing into the bioreactor vessel (30) on the supported 2D cell layer (20)
- an organoid (10) containing cells of at least two further cell types which are arranged in a defined 3D structure relative to each other, and
- a hydrogel (15)
with the proviso that the organoid (10) in the bioreactor vessel (30) is defined spaced apart from the supported 2D cell layer (20) by the introduced hydrogel (15).

2. The method according to claim 1, wherein in step (c) the further proviso applies that the organoid (10) in the bioreactor vessel (30) is also spaced apart from the walls (38) of the bioreactor vessel (30) by the introduced hydrogel (15).

3. The method of claim 1 or 2, wherein in step (c) the organoid (10) is introduced into the bioreactor vessel (30) together with the hydrogel (15).

4. The method of claim 1 or 2, wherein step (c) contains the substeps of:
(c1) introducing a part of the hydrogel to form a defined spacer layer (18) relative to the 2D cell layer (20) and optionally relative to the walls (38) of the bioreactor vessel (30), and then
(c2) introducing the organoid (10) on the hydrogel spacer layer (18) formed.

5. The method according to any one of the preceding claims, wherein in the cultivation of the 2D cell layer (20) on the bottom semipermeable membrane (33) at the basal pole (24) thereof is perfused separately from the apical pole (22) thereof.

6. An in vitro tissue culture in a bioreactor vessel (30) with a semipermeable membrane (33) on the bottom, containing:
- a 2D cell layer (20) containing at least a first cell type on the semipermeable membrane (33),
- an organoid (10) containing cells of at least two further cell types which are arranged in a defined 3D structure relative to each other, and
- a hydrogel (15) in which the organoid (10) in the bioreactor vessel (30) is embedded and which is spaced apart from the bottom 2D cell layer (20) by a defined distance.

7. The in vitro tissue culture according to claim 6, wherein the defined distance of the organoid (10) to the 2D cell layer (20) is 1 to 100 µm, preferably 2 to 20 µm.

8. The in vitro tissue culture according to claim 6 or 7, wherein the organoid (10) is embedded in the hydrogel (15) so that said organoid is also spaced apart from the walls (38) of the bioreactor vessel (30).

9. The in vitro tissue culture according to one of claims 6 to 8, wherein a first 2D cell layer (20) is disposed on top of the membrane (33) facing the organoid (10).

10. The in vitro tissue culture according to claim 9, wherein a further 2D cell layer (25) is arranged on the bottom of the membrane (33) facing away from the organoid (10).

11. The in vitro tissue culture according to one of claims 6 to 10, wherein the cell type of the 2D cell layer (20, 25) is selected from:
- epithelial cells,
- epithelial-like cells,
- endothelial cells,
- stromal cells containing fibrocytes and/or fibroblasts, and
- muscle cells containing myoblasts, myocytes and/or muscle fibers.

12. The in vitro tissue culture according to one of claims 9 to 11, wherein the first 2D cell layer (20) on the top of the membrane (33) facing the organoid (10) contains or consists of epithelial cells.

13. The in vitro tissue culture according to claim 12, wherein the further 2D cell layer (25) on the bottom of the membrane (33) facing away from the organoid (10) contains or consists of endothelial cells.

14. The in vitro tissue culture according to one of claims 6 to 13, wherein the organoid (10) is selected from the group of self-organizing multi-cell type tissues or multi-cell type tissues with defined 3D structures which can be produced by cell pressure, containing: retinal organoids, brain organoids, pancreatic organoids, and intestinal organoids.

15. The in vitro tissue culture according to one of claims 6 to 14, wherein the organoid (10) is a retina organoid which contains at least photoreceptor cells and cells of at least one other cell type of the neural vertebrate retina, and wherein the 2D cell layer (20) is a confluent monolayer of retinal pigment epithelial cells.

## Revendications

1. Procédé pour la co-culture microphysiologique de tissu organoïde (10) dans un récipient de bioréacteur (30) avec une membrane semi-perméable (33) sur le fond, comprenant les étapes consistant à :
(a) ensemencer des cellules d'au moins un premier type de cellule sur la membrane (33),
(b) cultiver ces cellules ensemencées pour former au moins une couche cellulaire 2D (20) supportée par cette membrane (33) et
(c) introduire dans le récipient de bioréacteur (30), sur la couche cellulaire 2D supportée (20)
- un organoïde (10) contenant des cellules d'au moins deux autres types de cellules qui sont disposées les unes par rapport aux autres dans une structure 3D définie, et
- un hydrogel (15)
à condition que l'organoïde (10) dans le récipient de bioréacteur (30) soit espacé de manière définie de la couche cellulaire 2D supportée (20) par l'hydrogel introduit (15).

2. Procédé selon la revendication 1, dans lequel, à l'étape (c), la condition supplémentaire est que l'organoïde (10) dans le récipient de bioréacteur (30) est supplémentaire également espacé des parois (38) du récipient de bioréacteur (30) par l'hydrogel introduit (15).

3. Procédé selon la revendication 1 ou 2, dans lequel, à l'étape (c), l'organoïde (10) est introduit dans le récipient de bioréacteur (30) avec l'hydrogel (15).

4. Procédé de la revendication 1 ou 2, dans lequel l'étape (c) contient les sous-étapes de :
(c1) introduire une partie de l'hydrogel pour former une couche d'espacement définie (18) par rapport à la couche cellulaire 2D (20) et éventuellement par rapport aux parois (38) du récipient de bioréacteur (30), puis
(c2) introduire l'organoïde (10) sur la couche d'espacement d'hydrogel (18) formée.

5. Procédé selon l'une quelconque des revendications précédentes, dans lequel lors de la culture de la couche cellulaire 2D (20) sur la membrane semi-perméable inférieure (33) au pôle basal (24) de celle-ci est perfusée séparément du pôle apical (22) de celle-ci.

6. Culture de tissu in vitro dans un récipient de bioréacteur (30) avec une membrane semi-perméable (33) sur le fond, contenant :
- une couche cellulaire 2D (20) contenant au moins un premier type de cellules sur la membrane semi-perméable (33),
- un organoïde (10) contenant des cellules d'au moins deux autres types de cellules qui sont disposées les unes par rapport aux autres dans une structure 3D définie, et
- un hydrogel (15) dans lequel l'organoïde (10) est incorporé dans le récipient de bioréacteur (30) et qui est espacé de la couche cellulaire 2D (20) inférieure d'une distance définie.

7. Culture de tissu in vitro selon la revendication 6, dans laquelle la distance définie entre l'organoïde (10) et la couche cellulaire 2D (20) est de 1 à 100 µm, de préférence de 2 à 20 µm.

8. Culture de tissu in vitro selon la revendication 6 ou 7, dans laquelle l'organoïde (10) est incorporé dans l'hydrogel (15) de sorte que ledit organoïde est également espacé des parois (38) du récipient de bioréacteur (30).

9. Culture de tissu in vitro selon l'une des revendications 6 à 8, dans laquelle une première couche cellulaire 2D (20) est disposée sur le dessus de la membrane (33) tournée vers de l'organoïde (10).

10. Culture de tissu in vitro selon la revendication 9, dans laquelle une autre couche cellulaire 2D (25) est disposée sur le bas de la membrane (33) tournée vers le contraire de l'organoïde (10).

11. Culture de tissu in vitro selon l'une des revendications 6 à 10, dans laquelle le type de cellule de la couche cellulaire 2D (20, 25) est choisi parmi :
- les cellules épithéliales,
- les cellules de type épithélial,
- les cellules endothéliales,
- les cellules stromales contenant des fibrocytes et/ou des fibroblastes, et
- les cellules musculaires contenant des myoblastes, des myocytes et/ou des fibres musculaires.

12. Culture de tissu in vitro selon l'une des revendications 9 à 11, dans laquelle la première couche cellulaire 2D (20) sur le dessus de la membrane (33) tournée vers l'organoïde (10) contient ou est constituée de cellules épithéliales.

13. Culture de tissu in vitro selon la revendication 12, dans laquelle l'autre couche cellulaire 2D (25) sur le bas de la membrane (33) tournée vers le contraire de l'organoïde (10) contient ou consiste en des cellules endothéliales.

14. Culture de tissu in vitro selon l'une des revendications 6 à 13, dans laquelle l'organoïde (10) est choisi dans le groupe des tissus de type multi-cellulaire auto-organisés ou des tissus de type multi-cellulaire avec des structures 3D définies qui peuvent être produites par pression cellulaire, contenant : des organoïdes rétiniens, des organoïdes cérébraux, des organoïdes pancréatiques et des organoïdes intestinaux.

15. Culture de tissu in vitro selon l'une des revendications 6 à 14, dans laquelle l'organoïde (10) est un organoïde rétiniens qui contient au moins des cellules photoréceptrices et des cellules d'au moins un autre type cellulaire de la rétine des vertébrés neuraux, et dans laquelle la couche cellulaire 2D (20) est une monocouche confluente de cellules épithéliales du pigment rétinien.
